# EUROPÄISCHE PATENTSCHRIFT

(11) **EP 2 723 704 B1**
(45) Veröffentlichungstag und Bekanntmachung des Hinweises auf die Patenterteilung: **29.06.2016**
(21) Anmeldenummer: 12722124.0
(22) Anmeldetag: 15.05.2012
(51) Int. Cl.: C07C 51/09, C07C 53/128, C07C 67/08

(54) **VERFAHREN ZUR HERSTELLUNG VON 2-ETHYLHEPTANSÄURE**
METHOD FOR THE PRODUCTION OF 2-ETHYLHEPTANOIC ACID
PROCÉDÉ DE PRODUCTION D'ACIDE 2-ÉTHYLHEPTANOÏQUE

(30) Priorität: 21.06.2011 DE 102011077860
(43) Veröffentlichungstag der Anmeldung: 30.04.2014
(73) Patentinhaber: Evonik Degussa GmbH, 45128 Essen (DE)
(72) Erfinder: BÖING, Christian, 50679 Köln (DE); MASCHMEYER, Dietrich, 45657 Recklinghausen (DE); WINTERBERG, Markus, 45711 Datteln (DE); GRASS, Michael, 45721 Haltern am See (DE); GEVERS, Andreas, 46240 Bottrop (DE); BECKER, Hinnerk Gordon, 45257 Essen (DE)
(86) Internationale Anmeldenummer: PCT/EP2012/059011
(87) Internationale Veröffentlichungsnummer: WO 2012/175255

(56) Entgegenhaltungen:
- WO-A1-02/16301
- WO-A1-2008/084046
- DE-A1- 2 003 522
- DE-A1- 2 306 405
- DE-A1-102007 006 442
- US-A1- 2003 116 750
- BEHR, A.; BREHME, V.A.: "Bimetallic-Catalyzed reduction of Carboxylic Acids and Lactones to Alcohols and Diols", ADVNANCED SYNTHESIS AND CATALYSIS, Bd. 344, Nr. 5, 2002, Seiten 525-532, XP002682254,
- A. BEHR; V.A. BREHME: JOURNAL OF MOLECULAR CATALYSIS A: CHEMICAL, Bd. 187, 2002, Seiten 69-80, XP002682255, in der Anmeldung erwähnt

## Beschreibung

Die vorliegende Erfindung betrifft ein Verfahren zur Herstellung von 2-Ethylheptansäure.

Die 2-Ethylheptansäure ist an sich bekannt. Sie weist Eigenschaften auf, die sie als Ersatz für die bis jetzt in großem Maßstab eingesetzte 2-Ethylhexansäure prädestinieren.

Darüber hinaus besitzen die mit 2-Ethylheptansäure hergestellten Weichmacher aufgrund der höheren Kohlenstoffzahl eine geringere Flüchtigkeit.

Bisher ist der Ersatz von 2-Ethylhexansäure durch 2-Ethylheptansäure jedoch nicht gelungen, da kein Herstellprozess bekannt ist, der 2-Ethylheptansäure in hohen Ausbeuten, kostengünstig und in für den großtechnischen Maßstab geeigneter Weise produzieren kann.

Stand der Technik ist ein Herstellprozess, der in WO 02/16301 beschrieben wird. Hier wird das Lacton 2-Ethyliden-6-hepten-5-olid zu 2-Ethylidenhexensäure gespalten und anschließend zu 2-Ethylheptansäure hydriert. Der Ausgangsstoff 2-Ethyliden-6-hepten-5-olid wird in einem bekannten Verfahren aus 2 Molekülen 1,3-Butadien sowie einem Molekül Kohlendioxid kostengünstig hergestellt (das Verfahren wird beschrieben in Behr und Becker, Dalton Trans. 2006, 4607-4613).

Der Schlüsselschritt des dort beschriebenen Verfahrens ist die Spaltung des 2-Ethyliden-6-hepten-5-olid, wobei die hydrierende Spaltung des Lactons mit Hilfe von "Single Site"-Komplexen von Metallen der 8. Nebengruppe, die mit Phosphinliganden modifiziert sind, katalysiert wird. Das Verfahren wird mittels eines homogen gelösten Katalysators durchgeführt. Alternativ werden die Immobilisierung des "Single Site"-Katalysators auf einem festen Träger sowie die Anwendung eines Flüssig-Flüssig-2-Phasen-Systems in dem eine Phase aus Wasser besteht als Reaktionsführung vorgeschlagen. Bei der Immobilisierung des Katalysators auf einem festen Träger werden die Phosphinliganden so modifiziert, dass sie an den Träger gebunden werden können. Bei Anwendung eines Flüssig-Flüssig-2-Phasen-Systems, in dem eine Phase aus Wasser besteht, werden die Phosphinliganden mit Gruppen modifiziert, die eine Wasserlöslichkeit bewirken.

Nachteile des in WO 02/16301 beschriebenen Verfahrens sind
1. Aufwändige Herstellung der Katalysatoren
2. Hohe Empfindlichkeit der Katalysatoren gegenüber Sauerstoff und Wasser
3. Schwierige Katalysatorabtrennung bei homogener Reaktionsweise
4. Aufwändige Abwandlung der Phosphinliganden bei Immobilisierung bzw.

### kontinuierlicher Katalysatorabtrennung

Eine Aufgabe der vorliegenden Erfindung besteht darin, eine Verfahren zur Herstellung von 2-Ethylheptansäure zu entwickeln, das einen oder mehrere dieser oben genannten Nachteile nicht aufweist, und somit eine Verbesserung zu diesem Stand der Technik darstellt.

Die DE 2 003 522 beschreibt eine hydrogenolytische Spaltung von gesättigten Estern an bifunktionellen Katalysatoren auf Basis von Übergangsmetallen auf einem Träger mit Säurefunktionen, wobei es sich gemäß Erfindung entweder um lonentauscherharze oder kristalline, zeolithische Festkörpersäuren handelt. Besonders vorteilhaft wird gemäß dieser Druckschrift eine Festkörpersäure verwendet, die einen "Alkylierungsindex" über 100 besitzt. Dieser Index ist definiert als die Menge (in mmol Alkylat) an Propyltoluol, die an einem Gramm der Säure unter Standardbedingungen (100 °C, Propylen gesättigt) in der Stunde gebildet wird.

Dieses Verfahren kann nicht für die großtechnische Spaltung des 3,6-Diethyltetrahydro-pyran-2-on angewandt werden, aufgrund der völlig unbefriedigenden Standzeit des Katalysators.

In A. Behr, V.A. Brehme / Journal of Molecular Catalysis A: Chemical 187 (2002) 69-80 wird auf Seite 71 das Problem beschrieben, den Ring von 3,6-Diethyltetrahydro-2H-pyran-2-on, welches abgesehen von der Ketofunktion keine weiteren Doppelbindungen aufweist, zu öffnen. Das Problem konnte in oben genannten Artikel nicht gelöst werden. Somit bestand gegenüber 3,6-Diethyltetrahydro-2H-pyran-2-on das Vorurteil, dass genau bei dieser Verbindung keine Ringöffnungsreaktion durchgeführt werden kann.

Eine weitere Aufgabe der Erfindung bestand also darin, das Vorurteil, dass eine Ringöffnung für jene spezielle Verbindung, 3,6-Diethyltetrahydro-pyran-2-on, nicht möglich sei, zu überwinden, und eben für genau diese Verbindung eine solche Ringöffnungsreaktion zu entwickeln, welche sich auch noch im industriellen Maßstab umsetzen lassen könnte.

Die Aufgaben wurden gelöst durch ein Verfahren zur Herstellung von 2-Ethylheptansäure, umfassend den folgenden Verfahrensschritt: 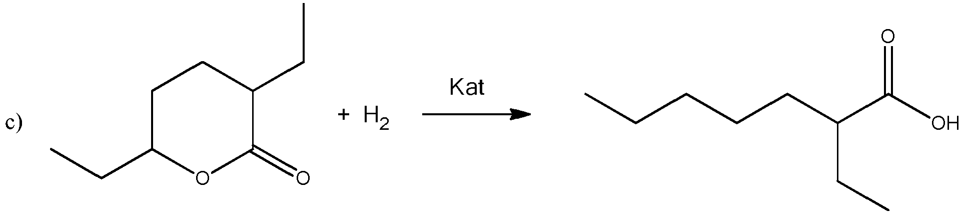 wobei der Verfahrensschritt c) heterogen-katalysiert an einem bifunktionellen Katalysator oder an einer Mischung von mindestens zwei Katalysatorkomponenten durchgeführt wird, wobei der bifunktionellen Katalysator oder im Fall der Mischung eine Katalysatorkomponente eine sauer wirkende Komponente aufweist, und die sauer wirkende Komponente eine feste oxidische Säure umfasst, welche einen Alkylierungsindex unter 100 aufweist, wobei die feste oxidische Säure einen Stoff umfasst ausgewählt aus: amorphes Silica-Alumina, Zirkondioxid, Titandioxid und wobei der Katalysator Palladium, Platin oder Nickel enthält.

Überraschender Weise wurde jetzt gefunden, dass die Spaltung des 3,6-Diethyltetrahydro-pyran-2-ons wesentlich besser mit einer sehr hohen Standzeit des Katalysators verläuft, sofern man als Festkörpersäure statt eines kristallinen Feststoffes eine amorphe Silica-Alumina verwendet, die einen Alkylierungsindex unter 100 besitzt.

In einer weiteren Ausführungsform der Erfindung wird das 3,6-Diethyltetrahydro-2H-pyran-2-on durch den vorgelagerten Verfahrensschritt b) erhalten:

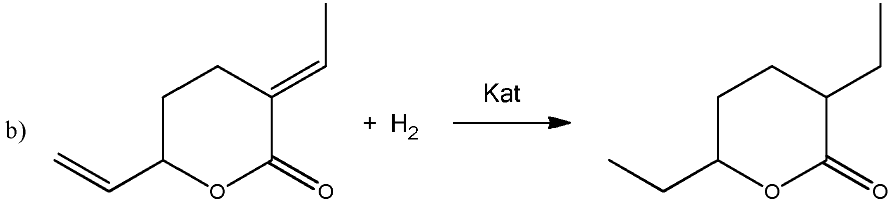

In einer weiteren Ausführungsform der Erfindung wird das 2-Ethyliden-6-hepten-5-olid durch den vorgelagerten Verfahrensschritt a) erhalten:

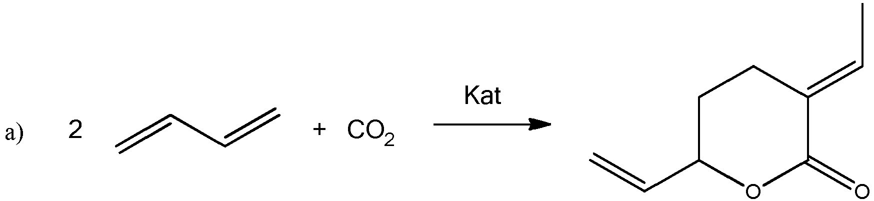

Die Herstellung von 2-Ethylheptansäure kann somit aus Butadien und CO₂ durch die Aneinanderreihung der folgenden Verfahrensschritte erfolgen:
a. Telomerisation von Butadien und CO₂ zu 2-Ethyliden-6-hepten-5-olid.
b. Hydrierung des 2-Ethyliden-6-hepten-5-olid zu 3,6-Diethyltetrahydro-pyran-2-on, vorzugsweise an einem heterogenen Katalysator auf neutralem Träger, beispielsweise Palladium auf alpha-Alumina.
c. Hydrogenolytische Spaltung des zu 3,6-Diethyltetrahydro-pyran-2-on zu 2-Ethylheptansäure.

Somit ist es nun möglich im großtechnischen Maßstab ausgehend von Butadien und CO₂ 2-Ethylheptansäure herzustellen.

Die feste oxidische Säure in Verfahrensschritt c) umfasst einen Stoff ausgewählt aus: amorphes Silica-Alumina, Zirkondioxid, Titandioxid. Hierbei ist das amorphe Silica-Alumina bevorzugt.

Der Verfahrensschritt c) des erfindungsgemäßen Verfahrens wird heterogen-katalysiert durchgeführt an einem Katalysator, der Palladium, Platin oder Nickel enthält, wobei Palladium besonders bevorzugt ist.

Der Katalysator kann technisch als bifunktioneller Katalysator ausgeführt sein oder aus einer Mischung von zwei Katalysatorkomponenten bestehen. Bevorzugt wird ein bifunktioneller Katalysator verwendet. Er kann derart hergestellt werden, dass die hydrierende Komponente auf die sauer wirkende Komponente aufgebracht wird. Dies kann durch Imprägnieren der sauer wirkenden Komponente mit einer Lösung eines Salzes der hydrierenden Komponente oder gemeinsamem Ausfällen von Salzen der sauer wirkenden Komponente und der hydrierenden Komponente durchgeführt werden, wie beschrieben in J. Hagen, Industrial Catalysis: A Practical Approach, Wiley-VCH, Weinheim, 2006. Bevorzugt wird eine Imprägnierung durchgeführt.

Der Verfahrensschritt c) des erfindungsgemäßen Verfahrens kann in flüssiger Phase, in flüssiger Phase plus gasförmiger Wasserstoffphase oder in der Gasphase durchgeführt werden. Bevorzugt wird er in flüssiger Phase unter dem Vorhandensein einer gasförmigen Wasserstoffphase durchgeführt. Bei dem Verfahrensschritt kann ein organisches Lösungsmittel vorhanden sein oder die Reaktion in Abwesenheit eines Lösungsmittels durchgeführt werden. Sollte ein Lösungsmittel vorhanden sein, können beispielsweise Paraffine oder Ether als Lösungsmittel verwendet werden. Bevorzugt wird die Reaktion in Abwesenheit eines Lösungsmittels durchgeführt.

Die Hydrogenolyse des Lactons 3,6-Diethyltetrahydro-2H-pyran-2-on geschieht vorzugsweise so, dass als Produkt 2-Ethylheptansäure in hohen Ausbeuten entsteht. Die häufig bei der Hydrogenolyse von Lactonen entstehenden Alkohole und Diole sind im erfindungsgemäßen Verfahren unerwünscht und so soll deren Bildung minimiert werden.

Der Verfahrensschritt c) des erfindungsgemäßen Verfahrens kann bei Temperaturen im Bereich von 25 bis 400 °C, bevorzugt im Bereich von 150 bis 350 °C, durchgeführt werden.

Der Druck kann im Bereich von 10 bis 200 bar, bevorzugt im Bereich von 20 bis 100 bar, liegen.

Das Zugeben von Wasserstoff zum Reaktionsgemisch kann in fein verteilter Form und in solchen Mengen erfolgen, dass das stöchiometrische Verhältnis von Wasserstoff zum Edukt 3,6-Diethyltetrahydro-2H-pyran-2-on zwischen 2 und 1 liegt. Bevorzugt liegt das Verhältnis zwischen 1.5 und 1.1. Besonders bevorzugt liegt es zwischen 1.2 und 1.1, da ansonsten die Gefahr besteht, dass die Säuregruppe zum Alkohol hydriert oder das Produkt weiter hydrogenolytisch gespalten wird.

Das Verhältnis der Masse des Zulaufstroms in den Reaktor zur Masse des Katalysators pro Stunde Verweilzeit [MZulauf/(VKat*VWZ), mit VWZ = Verweilzeit], dem Fachmann als WHSV (Weight hour space velocity) bekannt, kann beim Verfahrensschritt c) des erfindungsgemäßen Verfahren im Bereich von 0.1 und 20 h⁻¹, bevorzugt im Bereich von 0.5 bis 5 h⁻¹ liegen.

Dem Verfahrensschritt c) kann der folgende Verfahrensschritt b) vorgelagert sein, bei dem das 3,6-Diethyltetrahydro-2H-pyran-2-on erhalten wird:

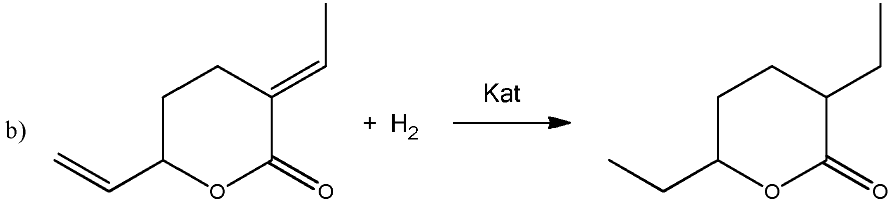

Das hier erhaltene 3,6-Diethyltetrahydro-2H-pyran-2-on kann in dem nachfolgenden Verfahrensschritt c) als Ausgangsstoff dienen.

Der Verfahrensschritt b) eines erfindungsgemäßen Verfahrens dieser Ausführungsform besteht aus der Hydrierung von 2-Ethyliden-6-hepten-5-olid zu 3,6-Diethyltetrahydro-2H-pyran-2-on. Dieser Verfahrenschritt wird vorzugsweise heterogen-katalysiert durchgeführt. Beispielsweise enthält der Katalysator in Verfahrensschritt b) Palladium, Platin oder Nickel. Auch Gemische dieser Metalle können verwendet werden.

Die Metalle können ohne Trägermaterialien oder mit Trägermaterialen eingesetzt werden. Sollte ein Trägermaterial verwendet werden, so können Trägermaterialen ausgewählt aus der Gruppe Aktivkohle, Aluminiumoxid, Siliziumoxid, Titanoxid, Zirkoniumoxid oder Magnesiumoxid verwendet werden. Bevorzugt wird Aktivkohle oder Aluminiumoxid verwendet.

Die Hydrierung von 2-Ethyliden-6-hepten-5-olid kann in flüssiger Phase, in flüssiger Phase plus gasförmiger Wasserstoffphase oder in der Gasphase durchgeführt werden. Bevorzugt wird sie in flüssiger Phase unter dem Vorhandensein einer gasförmigen Wasserstoffphase durchgeführt. Bei dem Verfahrensschritt kann ein organisches Lösungsmittel vorhanden sein oder die Reaktion in Abwesenheit eines Lösungsmittels durchgeführt werden. Sollte ein Lösungsmittel vorhanden sein, können beispielsweise niedere Alkohole, Paraffine oder Ether als Lösungsmittel verwendet werden.

Die Reaktionstemperatur liegt beispielsweise im Bereich von 0 bis 100 °C, bevorzugt im Bereich von 20 bis 80 °C, besonders bevorzugt im Bereich von 30 bis 70 °C.

Der Druck liegt üblicherweise im Bereich von 2 bis 50 bar, bevorzugt im Bereich von 6 bis 30 bar, besonders bevorzugt im Bereich von 10 bis 25 bar.

Weiterhin kann dem Verfahrensschritt c) beziehungsweise dem Verfahrensschritt b) ein weiterer Verfahrenschritt, der Verfahrensschritt a) vorgelagert sein. In diesem wird 2-Ethyliden-6-hepten-5-olid durch folgende Reaktion erhalten: 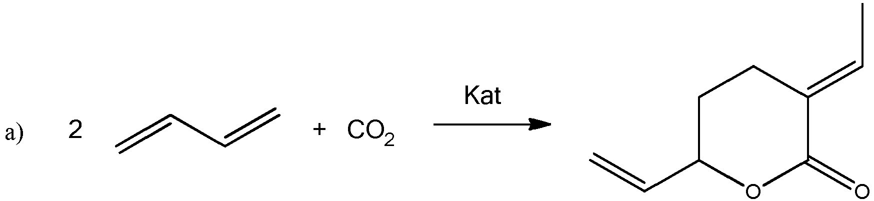 Das so erhaltene 2-Ethyliden-6-hepten-5-olid kann als Ausgangsstoff für den Verfahrensschritt b) dienen.

Das 2-Ethyliden-6-hepten-5-olid ist in hohen Ausbeuten durch eine Telomerisationsreaktion von zwei Molekülen 1,3-Butadien und einem Molekül Kohlendioxid kostengünstig zugänglich. Das Verfahren wird unter anderem in Behr und Becker, Dalton Trans. 2006, 4607-4613 beschrieben.

Die im Verfahrensschritt c) erhaltene 2-Ethylheptansäure wird in einer Ausführungsform des Verfahrens in einem weiteren Verfahrenschritt d) mit einem Alkohol zu einem Ester umgesetzt. Der Alkohol kann beispielsweise ausgewählt sein aus:
einwertige, zweiwertige, dreiwertige oder vierwertige Alkohole, besonders bevorzugt zweiwertige und dreiwertige Alkohole. Unter den zwei- oder dreiwertigen Alkoholen besonders bevorzugt sind beispielsweise Ethylenglycol, Diethylenglycol, Triethylenglycol, Tetraethylenglycol, Propylenglycol, Dipropylenglycol, Tripropylenglycol, Neopentylglycol, Isosorbid, Isomannid, Isoidid, Furan-2,5-dihydroxymethanol, Trimethylolpropan, Glycerin. Ganz besonders bevorzugt seien Isosorbid und Glycerin genannt.

Der so erhaltene Ester kann beispielsweise als Weichmacher, insbesondere für PVC oder PVB, verwendet werden.

Die erfindungsgemäß hergestellte 2-Ethylheptansäure kann neben der Verwendung als Ausgangsmaterial für Weichmacher auch vorteilhaft in Metallsalzen zum Einsatz als Thermostabilisator und Sikkativ sowie zur Herstellung von Schmierstoffen und zur Herstellung peroxidischer Verbindungen eingesetzt werden.

Die nachfolgenden Beispiele sollen die Erfindung näher erläutern, ohne dass deren Ausführung auf die in den Beispielen genannte Arbeitsweise beschränkt ist.

### Ausführungsbeispiele:

### Hydrierung von 2-Ethyliden-6-hepten-5-olid zu 3,6-Diethyltetrahydro-2H-pyran-2-on (Verfahrensschritt b))

Die Hydrierung wird in einem 3 Liter-Stahlautoklaven mit Heizmantel, durch den ein Wärmeträgeröl (Marlotherm SH der Sasol Olefins & Surfactants GmbH) floss, durchgeführt. Als Katalysator werden 5 g eines Schalenkatalysators mit 0.5% Palladium auf alpha-Aluminiumoxid in Kugelform verwendet, der in einem Käfig in den Reaktor so eingebaut ist, dass er von der gemischten Gas- und Flüssigphase optimal durchströmt wird.

152 g (1 mol) 2-Ethyliden-6-hepten-5-olid werden in den Stahlautoklaven eingefüllt und in 1 L Tetrahydrofuran gelöst. Anschließend wurde der Autoklav verschlossen. Durch Aufpressen von Wasserstoff wird ein Druck von 20 bar eingestellt. Diese Bedingungen werden bei 60 °C wird die Suspension für 20 h gehalten. Anschließend wird entspannt, die Flüssigphase abgezogen, das Lösungsmittel am Rotationsverdampfer abdestilliert und das Produkt durch

fraktionierte Destillation gereinigt. Die Ausbeute an 3,6-Diethyltetrahydro-2H-pyran-2-on betrug 145 g (93%).

Hydrogenolyse von 3,6-Diethyltetrahydro-2H-pyran-2-on zu 2-Ethylheptansäure

### (Verfahrensschritt c))

Die Hydrogenolyse wird in einem Rohrreaktor (Stahl 1.4571, Innenmaß 800 x 8 mm) mit Heizmantel, durch den ein Wärmeträgeröl (Marlotherm SH der Sasol Olefins & Surfactants GmbH) floss, durchgeführt. Als Katalysator werden 50 g Pellets (ca. 90 ml) aus amorpher Silica-Alumina mit 13 % Aluminiumoxidgehalt und einer BET-Oberfläche von 290 m2/g verwendet, die mit 2,0 % Palladium imprägniert sind. Die Reaktionstemperatur beträgt 270 °C. Der WHSV-Wert bezogen auf 3,6-Diethyltetrahydro-2H-pyran-2-on beträgt 0,8 kg/l/h bezogen auf den leeren Reaktor. Parallel zu 3,6-Diethyltetrahydro-2H-pyran-2-on werden 15 Nl/h Wasserstoff in den Zulauf des Reaktors geleitet. Der Reaktor wird mit Argon auf einem Druck von 30 bar gehalten. Der Austrag des Reaktors wird gaschromatographisch analysiert. Die Ausbeute beträgt 89% an 2-Ethylheptansäure.

## Patentansprüche

1. Verfahren zur Herstellung von 2-Ethylheptansäure, umfassend den folgenden Verfahrensschritt: 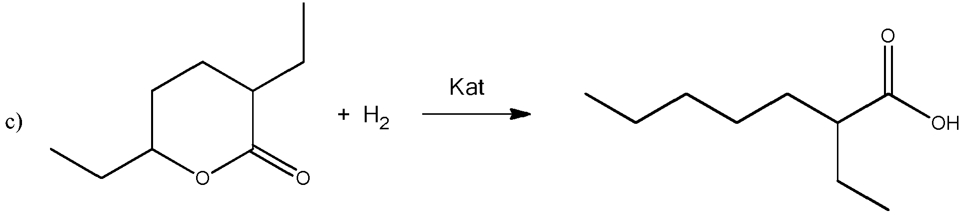 wobei der Verfahrensschritt c) heterogen-katalysiert an einem bifunktionellen Katalysator oder an einer Mischung von mindestens zwei Katalysatorkomponenten durchgeführt wird, wobei der bifunktionellen Katalysator oder im Fall der Mischung eine Katalysatorkomponente eine sauer wirkende Komponente aufweist, und die sauer wirkende Komponente eine feste oxidische Säure umfasst, welche einen Alkylierungsindex unter 100 aufweist, wobei die feste oxidische Säure einen Stoff umfasst ausgewählt aus: amorphes Silica-Alumina, Zirkondioxid, Titandioxid und wobei der Katalysator Palladium, Platin oder Nickel enthält.

2. Verfahren nach Anspruch 1, wobei das 3,6-Diethyltetrahydro-2H-pyran-2-on durch den vorgelagerten Verfahrensschritt b) erhalten wird:

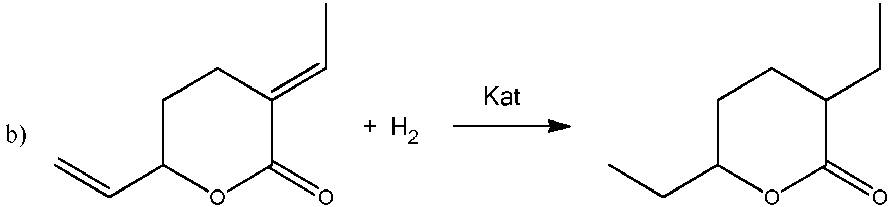

3. Verfahren nach einem der Ansprüche 1 oder 2, wobei das 2-Ethyliden-6-hepten-5-olid durch den vorgelagerten Verfahrensschritt a) erhalten wird:

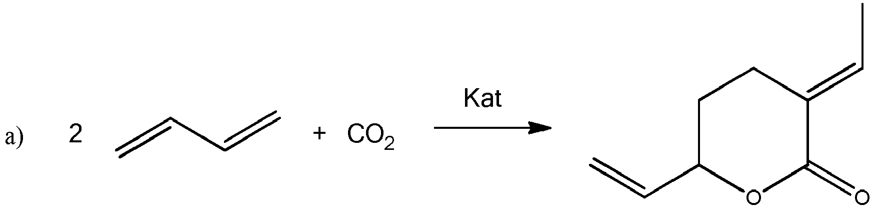

4. Verfahren nach einem der Ansprüche 1 bis 3,
wobei der Verfahrensschritt c) in Abwesenheit eines Lösungsmittels durchgeführt wird.

5. Verfahren nach einem der Ansprüche 1 bis 4,
wobei der Verfahrensschritt c) bei Temperaturen im Bereich von 25 °C bis 400 °C durchgeführt wird.

6. Verfahren nach einem der Ansprüche 1 bis 5,
wobei der Verfahrensschritt c) bei einem Druck im Bereich von 1 bar bis 200 bar durchgeführt wird.

7. Verfahren nach einem der Ansprüche 1 bis 6,
wobei der Verfahrensschritt c) bei einem Verhältnis der Masse des Zulaufstroms in den Reaktor zur Masse des Katalysators pro Stunde Verweilzeit aus dem Bereich von 0.1 und 20 h⁻¹ durchgeführt wird.

8. Verfahren nach einem der Ansprüche 2 bis 7,
wobei der Katalysator in Verfahrensschritt b) Palladium, Platin oder Nickel enthält.

9. Verfahren nach einem der Ansprüche 2 bis 8,
wobei der Verfahrensschritt b) bei Temperaturen im Bereich von 0 °C bis 100 °C durchgeführt wird.

10. Verfahren nach einem der Ansprüche 2 bis 9,
wobei der Verfahrensschritt b) bei einem Druck im Bereich von 2 bar bis 50 bar durchgeführt wird.

11. Verfahren nach einem der Ansprüche 1 bis 10,
wobei die im Verfahrensschritt c) erhaltene 2-Ethylheptansäure in einem weiteren Verfahrensschritt d) mit einem Alkohol zu einem Ester umgesetzt wird.

## Claims

1. Process for producing 2-ethylheptanoic acid, involving the following process step: 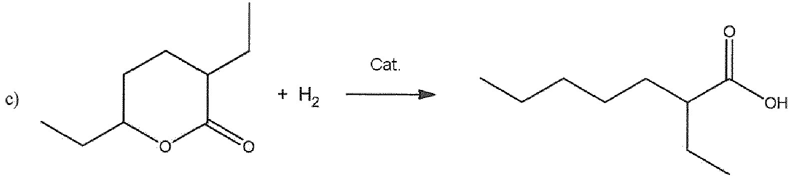 where process step c) is carried out with heterogeneous catalysis over a bifunctional catalyst or over a mixture of at least two catalyst components, where the bifunctional catalyst or, in the case of the mixture, one catalyst component has an acidically acting component, and the acidically acting component comprises a solid oxidic acid which has an alkylation index below 100, where the solid oxidic acid comprises a substance selected from: amorphous silica-alumina, zirconium dioxide, titanium dioxide and where the catalyst comprises palladium, platinum or nickel.

2. Process according to Claim 1,
where the 3,6-diethyltetrahydro-2H-pyran-2-one is obtained by the upstream process step b):

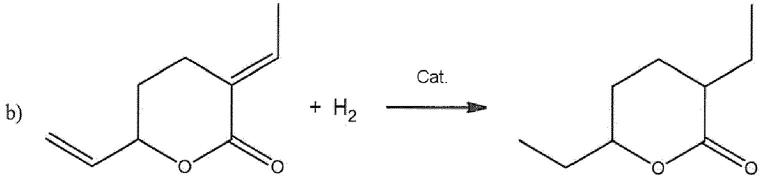

3. Process according to one of Claims 1 or 2,
where the 2-ethylidene-6-hepten-5-olide is obtained by the upstream process step a):

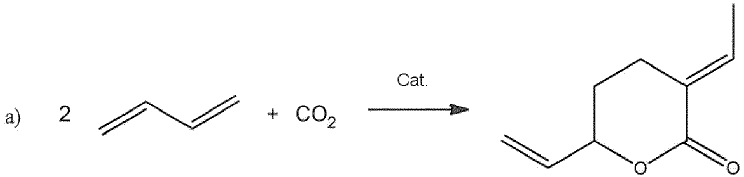

4. Process according to one of Claims 1 to 3,
where process step c) is carried out in the absence of a solvent.

5. Process according to one of Claims 1 to 4,
where process step c) is carried out at temperatures in the range from 25°C to 400°C.

6. Process according to one of Claims 1 to 5,
where process step c) is carried out at a pressure in the range from 1 bar to 200 bar.

7. Process according to one of Claims 1 to 6,
where process step c) is carried out at a ratio of the mass of the feed stream into the reactor to the mass of the catalyst per hour of residence time from the range from 0.1 and 20 h⁻¹.

8. Process according to one of Claims 2 to 7,
where the catalyst in process step b) comprises palladium, platinum or nickel.

9. Process according to one of Claims 2 to 8,
where process step b) is carried out at temperatures in the range from 0°C to 100°C.

10. Process according to one of Claims 2 to 9,
where process step b) is carried out at a pressure in the range from 2 bar to 50 bar.

11. Process according to one of Claims 1 to 10,
where the 2-ethylheptanoic acid obtained in the process step c) is reacted in a further process step d) with an alcohol to give an ester.

## Revendications

1. Procédé pour la préparation d'acide 2-éthylheptanoïque, comprenant l'étape de procédé suivante : 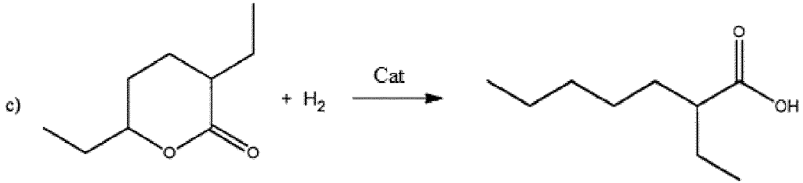 l'étape de procédé c) étant réalisée de manière catalysée par voie hétérogène sur un catalyseur difonctionnel ou sur un mélange d'au moins deux composants catalytiques, le catalyseur difonctionnel ou, dans le cas du mélange, un constituant catalytique présentant un composant à action acide et le composant à action acide comprenant un acide oxyde solide, qui présente un indice d'alkylation inférieur à 100, l'acide oxyde solide comprenant une substance choisie parmi : de la silice-alumine amorphe, du dioxyde de zirconium, du dioxyde de titane et le catalyseur contenant du palladium, du platine ou du nickel.

2. Procédé selon la revendication 1, le 3,6-diéthyltétrahydro-2H-pyran-2-one étant obtenu par l'étape de procédé b) disposée en amont :

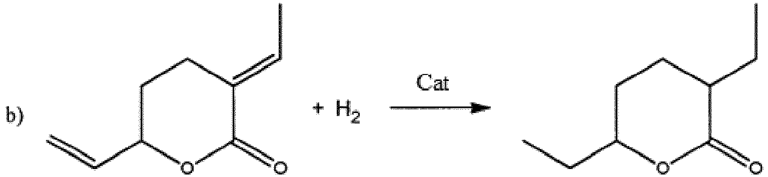

3. Procédé selon l'une quelconque des revendications 1 ou 2, le 2-éthylidène-6-heptén-5-olide étant obtenu par l'étape de procédé a) disposée en amont :

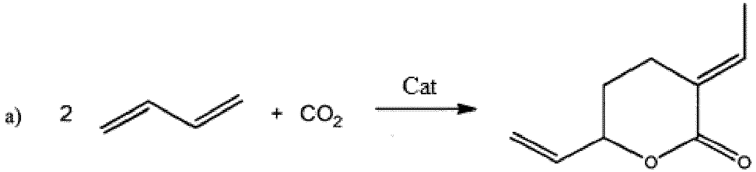

4. Procédé selon l'une quelconque des revendications 1 à 3, l'étape de procédé (c) étant réalisée en l'absence de solvant.

5. Procédé selon l'une quelconque des revendications 1 à 4, l'étape de procédé c) étant réalisée à des températures dans la plage de 25°C à 400°C.

6. Procédé selon l'une quelconque des revendications 1 à 5, l'étape de procédé c) étant réalisée à une pression dans la plage de 1 bar à 200 bars.

7. Procédé selon l'une quelconque des revendications 1 à 6, l'étape de procédé c) étant réalisée dans un rapport de la masse du flux d'alimentation dans le réacteur à la masse de catalyseur par heure de durée de séjour dans la plage de 0,1 à 20 h⁻¹.

8. Procédé selon l'une quelconque des revendications 2 à 7, le catalyseur utilisé dans l'étape de procédé b) contenant du palladium, du platine ou du nickel.

9. Procédé selon l'une quelconque des revendications 2 à 8, l'étape de procédé b) étant réalisée à des températures dans la plage de 0°C à 100°C.

10. Procédé selon l'une quelconque des revendications 2 à 9, l'étape de procédé b) étant réalisée à une pression dans la plage de 2 bars à 50 bars.

11. Procédé selon l'une quelconque des revendications 1 à 10, l'acide 2-éthylheptanoïque obtenu dans l'étape de procédé c) étant transformé dans une autre étape de procédé d) avec un alcool en ester.
